# EUROPEAN PATENT SPECIFICATION

(11) **EP 3 801 406 B1**
(45) Date of publication and mention of the grant of the patent: **07.08.2024**
(21) Application number: 19816177.0
(22) Date of filing: 07.06.2019
(51) Int. Cl.: A61F 2/95, A61F 2/06, A61F 2/86, A61F 2/90, A61F 2/958, A61F 2/966, A61B 1/06, A61B 1/05, A61B 1/018, A61B 1/00

(54) **ENDOSCOPE FOR PLACING AND REMOVING SURGICAL STENTS**
ENDOSKOP ZUM PLATZIEREN UND ENTFERNEN VON CHIRURGISCHEN STENTS
ENDOSCOPE POUR LE PLACEMENT ET RETRAIT DE STENTS CHIRURGICAUX

(30) Priority: 07.06.2018 US 201862681824 P; 07.06.2018 US 201862681687 P; 09.06.2018 US 201862682854 P; 19.06.2018 US 201862686680 P; 19.06.2018 US 201862686682 P; 22.06.2018 US 201862688397 P; 11.03.2019 US 201962816366 P; 25.03.2019 US 201916363209
(43) Date of publication of application: 14.04.2021
(73) Proprietor: Micronvision Corp, Bellevue WA 98006 (US)
(72) Inventor: OUYANG, Xiaolong, Bellevue, WA 98006 (US)
(74) Representative: Whitlock, Holly Elizabeth Ann
(86) International application number: PCT/US2019/036060
(87) International publication number: WO 2019/237003

(56) References cited:
- WO-A1-2014/065901
- US-A1- 2006 184 227
- US-A1- 2006 259 124
- US-A1- 2011 112 622
- US-A1- 2011 264 191
- US-A1- 2012 041 533
- US-A1- 2012 165 916
- US-A1- 2014 188 211
- US-A1- 2016 367 119
- US-B1- 6 211 904

## Description

### FIELD

This patent specification generally relates mainly to a medical device for Use in placing and retrieving surgical stents, for example, using endoscopy for placing and removing prostatic stents.

### BACKGROUND

There are many medical procedures for placing a stent inside a human body, such as blood vessels or urinary tracts. In the case of a prostatic stent, a stent is used to keep open the male urethra and allow the passing of urine in cases of prostatic obstruction and lower urinary tract symptoms. Conventional systems for placing any stents are believed to be cumbersome, inefficient and can cause local trauma and/or substantial patient discomfort. Many conventional stent placement procedures are carried out with medical equipment that is not specialized and/or customized for a particular application. For example, in the case of prostatic stents, it is common to deploy the stent using a complex, conventional, general purpose endoscopic device that has a rigid cannula, made of stainless steel, having an outer diameter of 7 mm or greater (21 fr.). Such systems are expensive, complicated and can cause significant discomfort and/or local trauma for the patient.

The subject matter described or claimed in this patent specification is not limited to embodiments that solve any specific disadvantages or that operate only in environments such as those described above. Rather, the above background is only provided to illustrate one exemplary technology area where some embodiments described herein may be practiced.

US 2012/0041533 A1 relates to methods and devices for imaging a body lumen during delivery and deployment of a medical device. A delivery device includes at least one sheath, a stent, an inner tubular member and at least one imaging device to allow visualization of the stent prior, during and after deployment without the use of an endoscope. The inner tubular member includes an articulating portion with the imaging device integrally formed and embedded therein.

WO 2014/065901 A1 relates to instruments and methods for performing endoscopically guided operative procedures. A re-usable portion of the instrument includes a handle, electronics and an integrated display screen while a fluid hub and a cannula which includes a CMOS imaging module and LED lighting, form a single use portion of the instrument. The cannula includes a working channel configured to accept an operative device for performing the operative procedures.

US 2011/0264191 A1 relates to a delivery system for delivery of an implantable stented device to a body lumen that includes an elongated member having a distal tip and a proximal end portion, a wire connection member positioned between the distal tip and proximal end portion of the elongated member, and a plurality of capturing wires extending from a distal end of the wire connection member. Each of the capturing wires includes a distal end having a lower portion that is moveable relative to an upper portion between an open position and a closed position, and a slot defined by the upper and lower portions when they are in the closed position.

US 2006/0184227 A1 relates to a system for treating a vascular condition and methods for assembling and using such a system. The system includes a catheter inner member, a stent framework, a graft material aligned with the stent framework to form a stent-graft assembly, and a slidable sheath. Multiple bands are spaced along the length of a distal portion of the inner member, each band at least partially encircling the inner member. The stent framework includes multiple rings. The stent-graft assembly is mounted on the inner member such that the stent frame-work rings are interspaced with the inner member bands. The movable sheath encloses the stent-graft assembly. As the sheath is withdrawn to deploy the stent-graft assembly, the interspaced bands and rings restrict axial movement of the assembly and prevent longitudinal compression or buckling of the assembly, as anchoring of the stent-graft assembly is distributed along the length of the inner member.

US 2014/0188211 A1 relates to an endoluminal prosthesis introducer having an elongate tubular sheath including a reinforced longitudinal segment and a peelable longitudinal segment extending distally from the reinforced segment. An elongate tubular cannula may be disposed within the sheath. Upon application of a force applied to a distal end of the sheath, the peelable segment may progressively split in a proximal direction, and the reinforced segment may longitudinally move relative to the cannula in a distal direction.

### SUMMARY

In the following, each of the described methods, apparatuses, embodiments, examples, and aspects, which do not fully correspond to the invention as defined in the claims is thus not according to the invention and is, as well as the whole following description, present for illustration purposes only or to highlight specific aspects or features of the claims. Embodiments not falling under the scope of the claims should be interpreted as examples useful for understanding the invention.

The invention relates to an endoscope according to independent claim 1.

The stent removal portion is configured to draw said stent proximally into said tubular space as the outer sleeve moves from its distal to its proximal positions.

Each of the single-use portion for deploying a stent and the single-use portion for removing a stent further comprises a housing 174 that has a proximal end connecting to said handle, a distal end connecting to said shaft, and an annular space in which a proximal portion of the shaft moves between said distal and proximal positions of the shaft, in some embodiments. The inner cannula is in a fixed position relative to the handle when said single-use and multiple-use portion are assembled into said endoscope, in some embodiments. The endoscope further includes proximal and distal ports in said outer sleeve and a fluid conduit through which fluid moves within and along a length of said outer sleeve between said ports, according to some embodiments. The inner cannula has a distal end stepped down in diameter to engage a matching diameter distal end of the stent to thereby prevent proximal motion but allow distal motion of the stent relative to the inner cannula, according to some embodiments. At least distal portions of the outer sleeve and the inner cannula are sufficiently flexible to bend when being inserted in curving portions of said passage, according to some embodiments.

According to some embodiment, an endoscope comprises: a handle 140 configured to be grasped by a user; a shaft 172 extending distally relative to the handle; an outer sleeve 422 extending distally from the shaft; and an inner cannula 420 configured to fit within the outer sleeve. The outer sleeve is configured to move relative to the handle and the inner cannula between a distal position and a proximal position. When the outer sleeve is in its proximal position a distal portion of the inner cannula protrudes distally from the outer sleeve and when the outer sleeve is in its distal position a tubular stent fits in a tubular space between distal portions of the outer sleeve and the inner cannula. A camera module 810 at the distal end of said inner cannula views the passage and a video screen 150 attached to and mechanically supported by said handle shows images of the passage and of the stent being deployed therein. The shaft, collar, outer sleeve and cannula form a single-use stent-deployment portion of the endoscope and the handle and video screen form a reusable portion. The two portions connect through connectors that mate and release by hand, without tools, to assemble the two portions into said endoscope and for disposal of said single-use portion after use thereof in a patient procedure.

For removing a stent from a passage in a patient, a single-use stent removal portion is configured to removably attach to said handle, in place of the stent deployment portion, and differs from said stent deployment portion by including plural hooks at a distal end of the inner cannula, which hooks are configured to fit between the inner cannula and the outer sleeve when the outer sleeve is in its distal position and to expand radially as the outer sleeve moves to its proximal position and to engage loops at a proximal portion of said stent. The stent removal portion is configured to draw said stent proximally into said tubular space as the outer sleeve moves from its distal to its proximal positions.

Each of the single-use portions further comprises a housing 174 that has a proximal end connecting to said handle, a distal end connecting to said shaft, and an annular space in which a proximal portion of the shaft moves between said distal and proximal position of the shaft, in some embodiments. Proximal and distal ports in said outer sleeve and a fluid conduit through which fluid moves within and along a length of said outer sleeve between said ports are provided, according to some embodiments. The inner cannula has a distal end stepped down in diameter to engage a matching diameter distal end of the stent to thereby prevent proximal motion but allow distal motion of the distal end of the stent relative to the inner cannula, according to some embodiments. At least distal portions of the outer sleeve and the inner cannula are sufficiently flexible to bend when being inserted in curving portions of said passage, according to some embodiments.

According to an example not according to the claimed invention, a method of deploying a stent in a passage in a patient and/or removing the stent comprises: placing a tubular stent in a compressed state in a tubular space between distal portions of an inner cannula and an outer sleeve of an endoscope such that the stent engages the inner cannula to prevent proximal motion but allow distal motion of the stent relative to the inner cannula; releasably securing said outer sleeve and inner cannula to a handle configured to be grasped by a user; inserting the outer sleeve in a passage in the patient's body, with the stent in said tubular space between the outer sleeve and the inner cannula; moving the outer sleeve proximally relative to the inner cannula, the stent, and the handle to thereby free the stent of being radially constrained by the outer sleeve and allow the stent to expand radially to an expanded state and remain in the passage in the patient; withdrawing the inner cannula from the patient; and imaging the passage with a video camera at a tip of the inner cannula while inserting the outer sleeve, moving the outer sleeve proximally relative to the inner cannula, and withdrawing the inner cannula, and displaying the images on a video screen secured to and mechanically supported by said handle. Moving the outer sleeve proximally includes moving a proximal portion of the sleeve within an annular space of a housing releasably secured to said handle, according to some embodiments. The method includes connecting said outer sleeve, inner cannula and housing to said handle through electrical and mechanical connectors that connect and disconnect by hand, without tools, according to some embodiments.

The method may further include removing a stent that has expanded radially and has remained in the passage by inserting an outer sleeve into the passage while viewing the passage with a video camera at a distal tip of an inner cannula that is in the outer sleeve, moving the outer sleeve proximally relative to the inner cannula to allow hook secured to the inner cannula to expand radially, engaging loops at a proximal portion of the stent with said hooks, moving one or both of the inner cannula and the outer sleeve relative to the other to draw the stent into a tubular space between distal portions of the outer sleeve and the inner cannula, and withdrawing the outer sleeve, the inner cannula, and the stent from the passage.

An alternative method, not covered by the invention, of removing a stent that has expanded radially and has remained in the passage comprises inserting an outer sleeve into the passage while viewing the passage with a video camera at a distal tip of an inner cannula that is in the outer sleeve, moving the outer sleeve proximally relative to the inner cannula to allow hook secured to the inner cannula to expand radially, engaging loops at a proximal portion of the stent with said hooks, and moving said inner cannula proximally relative to the passage to remove said stent from the passage.

As used herein, the grammatical conjunctions "and", "or" and "and/or" are all intended to indicate that one or more of the cases, object or subjects they connect may occur or be present. In this way, as used herein the term "or" in all cases indicates an "inclusive or" meaning rather than an "exclusive or" meaning.

As used herein the terms "surgical" or "surgery" refer to any physical intervention on a patient's tissues, and does not necessarily involve cutting a patient's tissues or closure of a previously sustained wound.

### BRIEF DESCRIPTION OF THE DRAWINGS

To further clarify the above and other advantages and features of the subject matter of this patent specification, specific examples of embodiments thereof are illustrated in the appended drawings. It should be appreciated that these drawings depict only illustrative embodiments and are therefore not to be considered limiting of the scope of this patent specification or the appended claims. The subject matter hereof will be described and explained with additional specificity and detail through the use of the accompanying drawings in which:
FIGs. 1, 2 and 3 are side, top and perspective views of an endoscopic system configured to deploy a surgical stent, according to some embodiments;
FIG. 4 is an exploded-view diagram of an endoscopic system configured to deploy a surgical stent, according to some embodiments;
FIGs. 5A-5C are diagrams illustrating the operation of deploying a surgical stent using an endoscopic system, according to some embodiments;
FIG. 5D is diagram illustrating a design for a sliding outer sleeve, according to some embodiments;
FIGs. 6A-6F are a series of diagrams illustrating a process of positioning and deploying a prostatic stent in a male urethra;
FIGs. 7A and 7B are side views of an endoscopically deployable surgical stent in compressed and expanded states, according to some embodiments;
FIG. 8 shows further detail of the distal tip of an endoscopic system configured to deploy a surgical stent, according to some embodiments;
FIGs. 9A and 9B are perspective views showing further detail of connectors between single use and multiple use portions of an endoscopic system configured to deploy a surgical stent, according to some embodiments;
FIG. 10 is a side view showing further detail of the distal tip of an endoscopic system configured to deploy a surgical stent, according to some embodiments;
FIGs. 11A-11C are diagrams illustrating further detail of a wire or rod used aid in deployment of a surgical stent, according to some embodiments;
FIGs. 12A-12F are various views of an endoscopic system configured to remove a surgical stent, according to some embodiments; and
FIGs. 13A-13F are a series of diagrams illustrating methods for removing a prostatic stent from a male urethra.

### DETAILED DESCRIPTION

A detailed description of examples of preferred embodiments is provided below. While several embodiments are described, it should be understood that the new subject matter described in this patent specification is not limited to any one embodiment or combination of embodiments described herein.

In addition, while numerous specific details are set forth in the following description in order to provide a thorough understanding, some embodiments can be practiced without some or all of these details. Moreover, for the purpose of clarity, certain technical material that is known in the related art has not been described in detail in order to avoid unnecessarily obscuring the new subject matter described herein. It should be clear that individual features of one or several of the specific embodiments described herein can be used in combination with features of other described embodiments or with other features. Further, like reference numbers and designations in the various drawings indicate like elements.

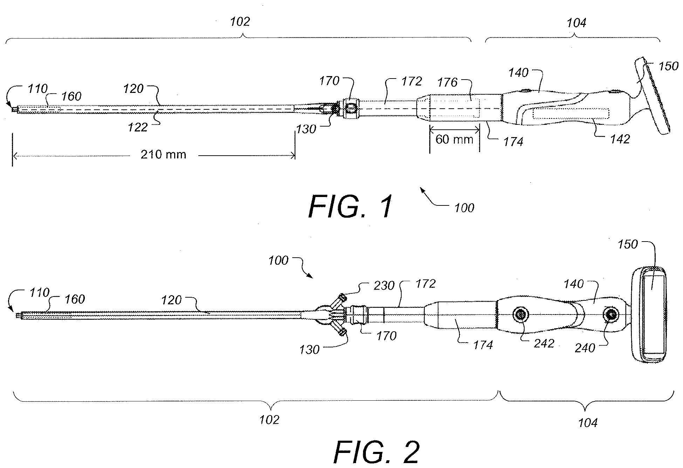

FIGs. 1, 2 and 3 are side, top and perspective views of an endoscopic system configured to deploy a surgical stent, according to some embodiments. System 100 is configured for minimally invasive and precise placement of a surgical stents. System 100 is adapted for easy and quick use with minimized patient discomfort and high placement accuracy. According to some embodiments, the system 100 includes a semi-rigid endoscope having an integrated stent 160 and dual cannula 120. Dual cannula 120 includes in inner cannula that has an imaging and illumination modules on its distal tip 110, and an outer sleeve. The outer sleeve is configured such that it can slide longitudinally with respect to the inner cannula. An electrical cable 122 is positioned within the inner cannula and supplies control signals and power to the camera and LED illumination modules on distal tip 110 and also transmits video image data from the camera module to the hand piece 140 and display 150 for viewing by an operator. In the example shown, hand piece 140 includes two control buttons 240 and 242 which can be configured for power on/off and image capture, respectively. According to some embodiments, hand piece 140 includes a rechargeable battery 142 as well as electronics or video capture, processing and display on or in display 150.

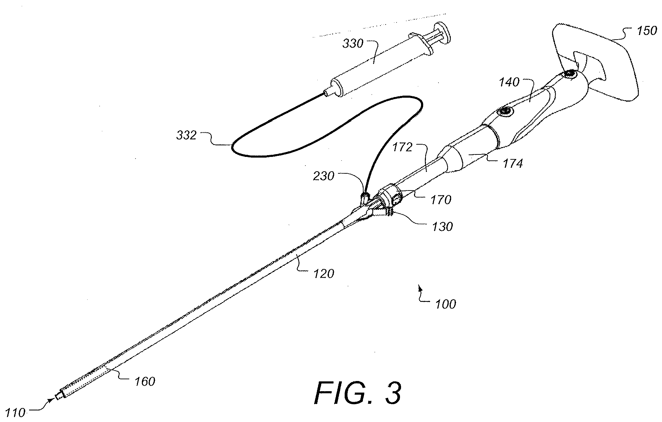

The cannula 120 is connected proximally to a fluid hub including in this example two fluid ports 130 and 230. Proximal to the fluid hub is a shaft 172 and a collar 170. According to some embodiments, the collar 170 is fixed to the outer sleeve of cannula 120 such that it can be used to retract and extend the outer sleeve relative to the inner cannula and stent 160 as will be shown and described further herein. Shaft 172 can slide into housing 174. Dashed outline 176 illustrates the position of shaft 172 in a retracted position within housing 174. When the operator moves collar 170 in the proximal direction, shaft 172 slides into housing 174 and along with it the outer sleeve of cannula 120 and fluid ports 130 and 230 move together. FIG. 3 show a syringe 330 used to supply fluid, such as saline, through a fluid lumen within cannula 120 via tubing 332 and fluid port 230. According to some embodiments, the system 100 is formed of a single use portion 102 and a multiple use portion 104. The portions 102 and 104 are connectable and separable via a mechanical and electrical connector. According to some embodiments the cannula 120 is semi-rigid. The cannula 120 is stiff enough so it does not collapse with the longitudinal pushing and pulling forces of positioning a stent. On the other hand, cannula 120 is flexible enough such that it can bend while it passes through curved anatomy such as male urethra.

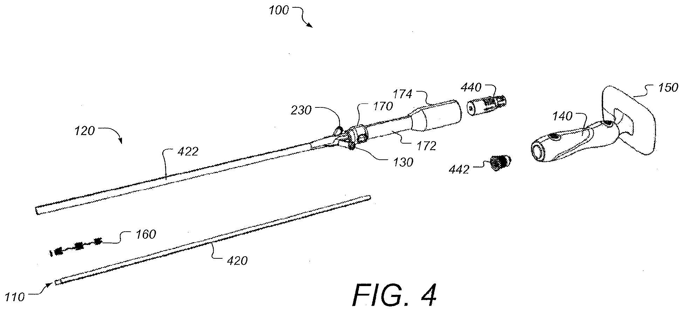

FIG. 4 is an exploded-view diagram of an endoscopic system configured to deploy a surgical stent, according to some embodiments. The inner cannula 420 and outer sleeve 422 of cannula 120 are shown. The inner cannula 420 and distal tip 110 reside within and slide relative to the outer sleeve 422. The stent 160, prior to deployment, is disposed in the annular region between the inner cannula 420 and the outer sleeve 422. Also shown is connector 440 that fits in the proximal end of shaft 172 on the single use portion, and connector 442 that fits in the distal end of hand piece 140. According to some embodiments, stent 160 is of a type that can be used to open up the urethra. The stent 160 can be made of nitinol. When expanded, the stent pushes back the surrounding tissue and widens the urethra. Outer sleeve 422 has a smooth exterior, and is made of Nylon or PTFE, and can be coated with a hydrophilic material to enhance its smoothness.

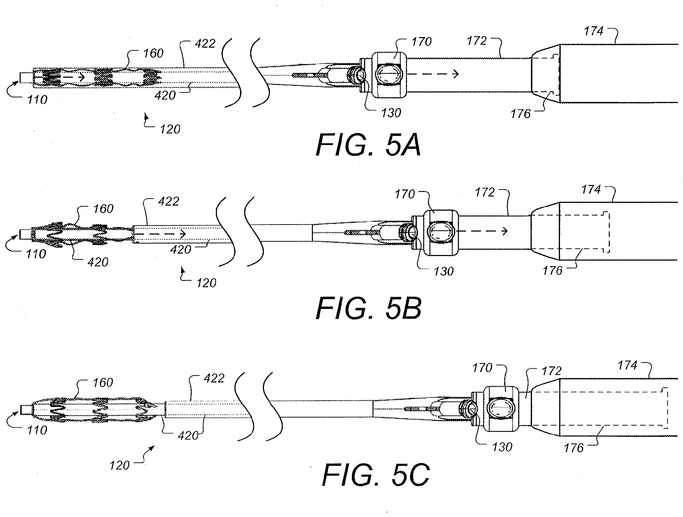

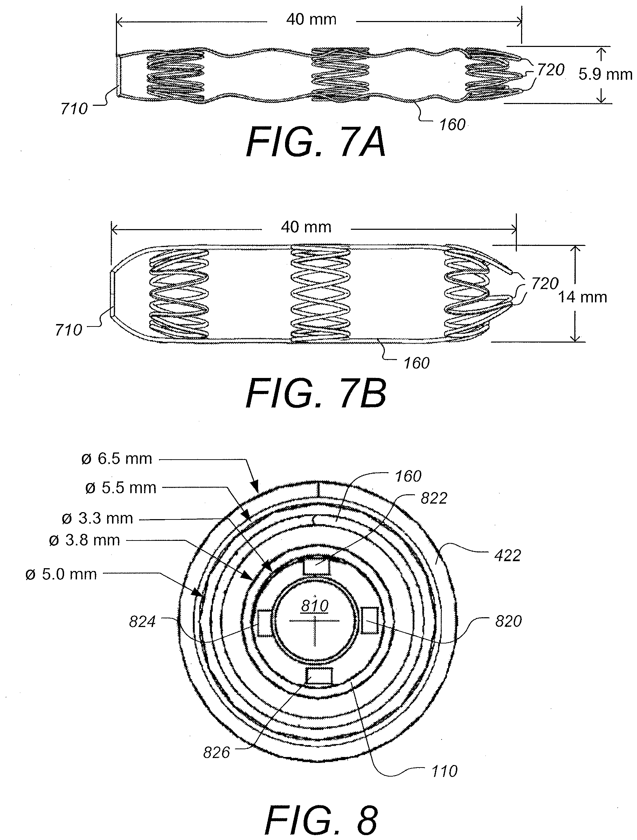

FIGs. 5A-5C are diagrams illustrating the operation of deploying a surgical stent using an endoscopic system, according to some embodiments. The cannula 120 has an outer sleeve 422 concentrically positioned around inner cannula 420. FIG. 5A shows the stent 160 in a compressed state positioned between the inner cannula 420 (shown in dotted outline) and the outer sleeve 422. The stent 160 is formed to be stable in an expanded state (shown in FIG. 7B) such that when it is compressed, as in the position shown in FIG. 5A, the stent 160 is secured by friction while it being held in the compressed stated by the inner surface of outer sleeve 422. Collar 170, shaft 172 and fluid port 130 are attached to the outer sleeve 422 such that those components slide longitudinally. As outer sleeve 422 is retracted proximally, a portion of shaft 172 moves into housing 174 as shown by the dashed outline 176. As shown in FIG. 5B, the collar 170 and shaft 172 are partially moved proximally (shown by the dashed arrows) and the outer sleeve 422 is retracted by the same longitudinal distance. On the other hand, the inner cannula 420 and distal tip 110 are fixed to housing 174 such that they do not move when the collar 170, shaft 172 and outer sleeve 422 are retracted proximally. The stent 160 is prevented from moving proximally either by friction alone or by a means of its distal tip fitting around a flange on the distal end of inner cannula 420. Alternatively or in addition, one or more radial projections from inner cannula 420 that are just proximal of stent 160 can help keep the stent from moving proximally along inner cannula 420. When the collar 170, shaft 172 and sleeve 422 are retracted proximally, as shown in FIG. 5B, the portion of stent 160 that is no longer being restrained by sleeve 422 is allowed to expand. FIG. 5C shows the collar 170, shaft 172 and sleeve 422 fully retracted proximally. In this position, no portion of the stent 160 is restricted by the outer sleeve 422 and the stent is free to expand into the available surrounding space.

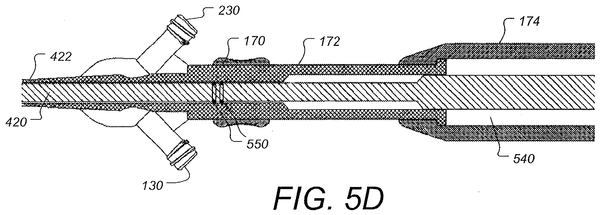

FIG. 5D is diagram illustrating a design for a sliding outer sleeve, according to some embodiments. As can be seen, collar 170, shaft 172 and fluid ports 130 and 230 are attached to the outer sleeve 422 such that those components slide longitudinally together. The shaft 172 fits inside of an annular space 540 within housing 174. According to some embodiments, o-rings 550 are provided to form a fluid seal which prevents fluid in the annular space between inner cannula 420 and outer sleeve 422 from leaking in a proximal direction.

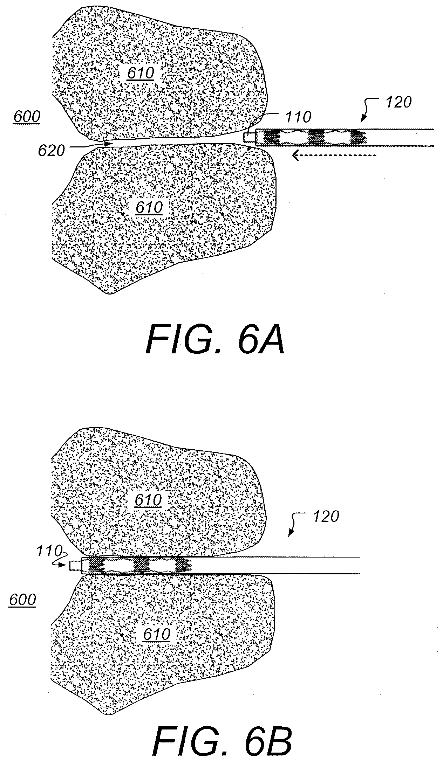

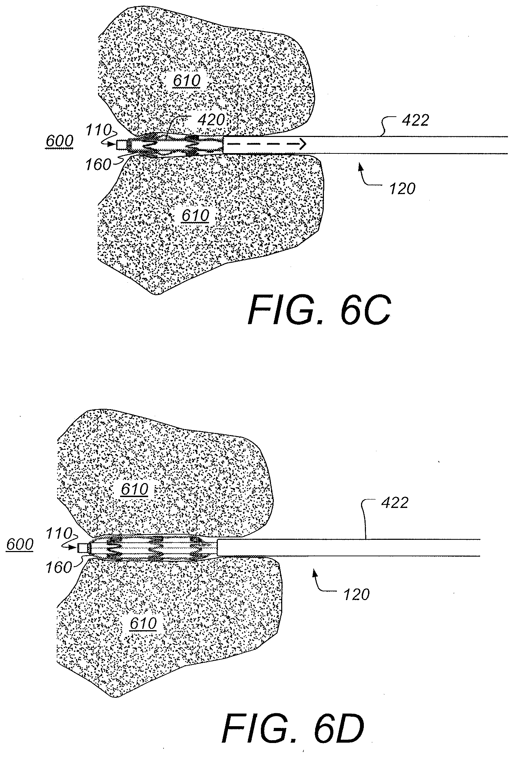

FIGs. 6A-6F are a series of diagrams illustrating a process of positioning and deploying a prostatic stent in a male urethra, according to some examples not according to the claimed invention. In FIG. 6A, under direct vision of live video captured by the camera module in distal tip 110 and displayed to the operator on display 150 (shown in FIGs. 1-4) the cannula 120 is slowly advanced through the urethra towards prostate 610 and bladder 600. In FIG. 6B, the cannula 120 is shown inserted
through the prostate canal 620 until the tip 110 reaches the entrance or neck of bladder. The precise location of the stent 160 with respect to the "neck" of bladder 600 and prostate canal 620 can be verified by the operator through the live images captured by the camera module in distal tip 110 and displayed to the operator on display 150 (shown in FIGs. 1-4). According to some embodiments, a distending media such as saline or CO₂ gas can flow through cannula 120 from either port 130 or 230 (e.g. shown in FIGs. 2 and 3) on the proximal end to the distal end, for example through the annular space between the inner cannula 420 and outer sleeve 422. In FIG. 6C, the outer sleeve 422 is slowly retracted while maintaining the longitudinal position of distal tip 110, inner cannula 420 and stent 160. This can be accomplished by the operator by maintaining the position of the hand piece 140 while slowly sliding the collar 170 proximally along shaft 172 (as shown in FIG. 5B). As the sleeve 422 is retracted, the portion of stent 160 that is no longer being restrained by sleeve 422 is allowed to expand against the walls of prostate canal 620. The sleeve 422 is retracted further until the distal end of the sleeve 422 is proximal to the proximal end of stent 160, as shown in FIG. 6D, such that the entire length of stent 160 is exposed.

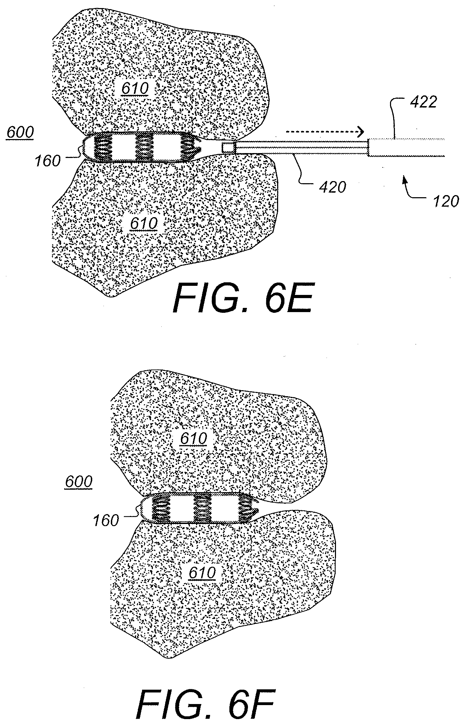

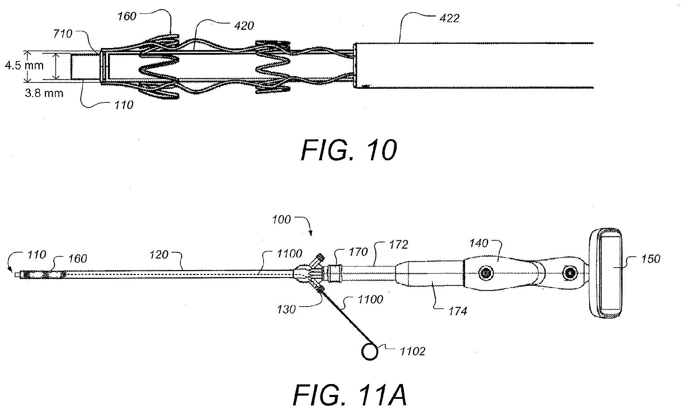

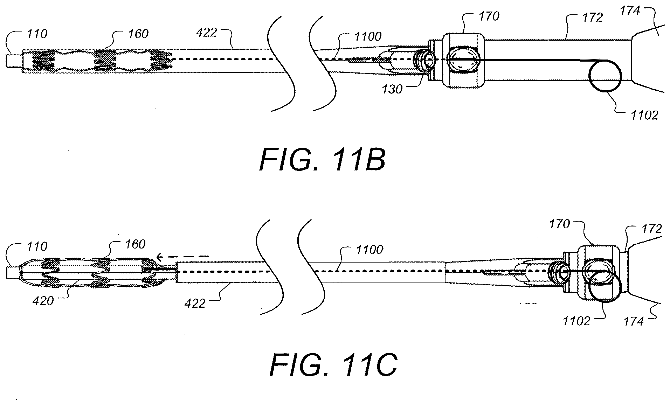

In FIG. 6E the cannula 120 is being withdrawn from the urethra as shown by the dotted arrow. The stent remains positioned in the prostate canal since it is pressed against the prostate canal wall. As the cannula 120 is slowly withdrawn, the correct positioning of the stent 160 can be verified by the operator through the live images captured by the camera module in distal tip 110 and displayed to the operator on display 150 (shown in FIGs. 1-4). The operator accomplishes this by sliding the collar 170 along shaft 172 to its most proximal position as shown in FIG. 5C. The stent 160 is now free to expand toward its stable expanded shape thereby exerting outward radial pressure on the prostate canal wall. According to some embodiments, a pusher wire or stick can be used, as shown in FIGs. 11A-11C to ensure release of the stent 160 from inner cannula 420 and to ensure proper positioning of stent 160. FIG. 6F shows the stent 160 expanded against the prostate canal wall after deployment.

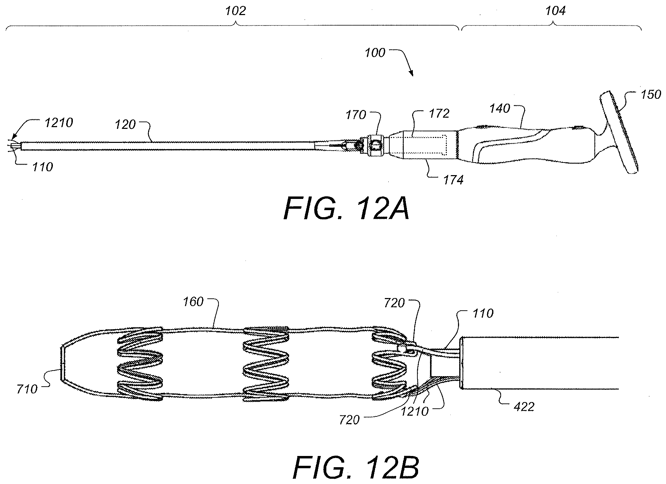

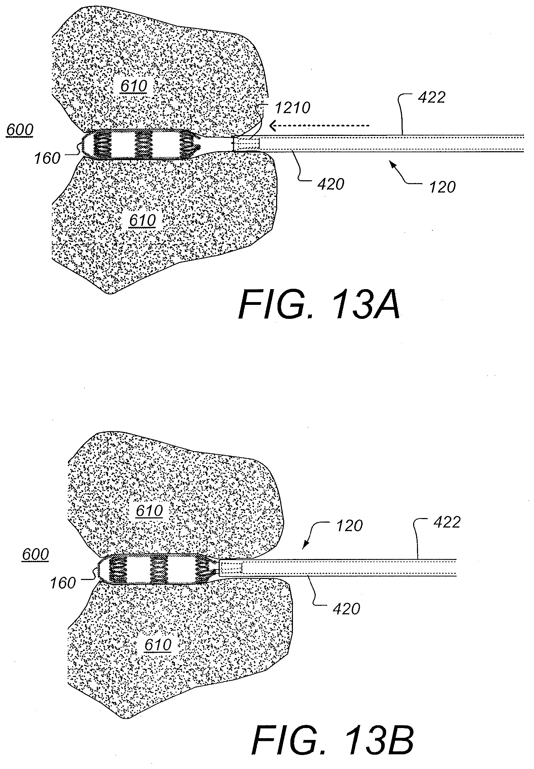

FIGs. 7A and 7B are side views of an endoscopically deployable surgical stent in compressed and expanded states, according some embodiments. FIG. 7A shows the example stent 160 in a compressed state, such as when it is positioned between the outer sleeve 422 and inner cannula 420 as shown in FIG. 5A. In this case the outer diameter of the stent is about 5.9 mm and the length is about 40 mm. FIG. 7B shows the example stent 160 in its expanded, relaxed, or stable state, such as shown in FIG. 5C. The length of the stent in its relaxed or expanded state is reduced to less than about 40 mm, for example to 20-30 mm, but the outer diameter is expanded to about 14mm. According to some embodiments, stent 160 includes a ring 710 that is dimensioned to fit over the outer surface of the distal tip 110, which is some cases is about 3.8 mm O.D. but is smaller than the outer diameter of the inner cannula, as shown in greater detail in FIG. 10. Also visible in FIGs. 7A and 7B are proximal loops 720 on stent 160. According to some embodiments, loops 720 are shaped such as to be engageable by a suitable stent retrieval tool, such as shown in greater detail in FIGs. 12A-B and 13A-13E.

FIG. 8 shows further detail of the distal tip of an endoscopic system configured to deploy a surgical stent, according to some embodiments. The stent 160 is shown positioned between the inner cannula and outer sleeve 422. The distal ring 710 of stent 160 is shown around the distal tip 110. The distal tip is shown to include camera module 810 and four LEDs 820, 822, 824 and 826 all of which fit within a distal tip housing having an inner diameter of 3.3 mm and outer diameter of 3.8 mm. According to some embodiments, camera module 180 has field of view (FOV) at least 120 degrees and a fixed focal range of about 3 mm. According to various other embodiments, the fixed focal range camera module 810 ranges from less than 3 mm to about 30mm. The outer sleeve 422 has an outer diameter of 6.5 mm and inner diameter of 5.5 mm. According to some embodiments, the outer diameter of the sleeve 422 can be made less than 6.5 mm. The stent 160 has a maximum outer diameter that matches (since it is constrained by) the inner diameter of sleeve 422. In the case shown in FIG. 8, the outer diameter of stent 160 is about 5.0 mm. According to some embodiments, the annular space or gap between the outer sleeve 422 and the inner cannula can be used for flow of distention fluid or gas. According to some embodiments, the outer sleeve 422 is formed of PTFE. The inner cannula 420 can also be formed of PTFE and can have an outer diameter of about 3.8 mm. The exterior of the outer sleeve can be coated with a hydrophilic material that becomes very slippery when wet during the medical procedure.

According to some embodiments, depending on the size of the camera module 810, the outer diameter of sleeve 422 can be made even less than 6.5 mm. In one example, the sleeve 422 has an outer diameter of 6.0 mm and an inner diameter of 4.9 mm. In this case the housing of distal tip 110 which includes camera module 810 and LEDs 820, 822, 824 and 826, has an inner diameter of 2.4 mm and outer diameter of 2.9 mm. The stent 160 in this case fits within the inner diameter of sleeve 422 and is has 4.0 mm outer diameter while compressed. While these dimensions are preferred, other embodiments can use other dimensions.

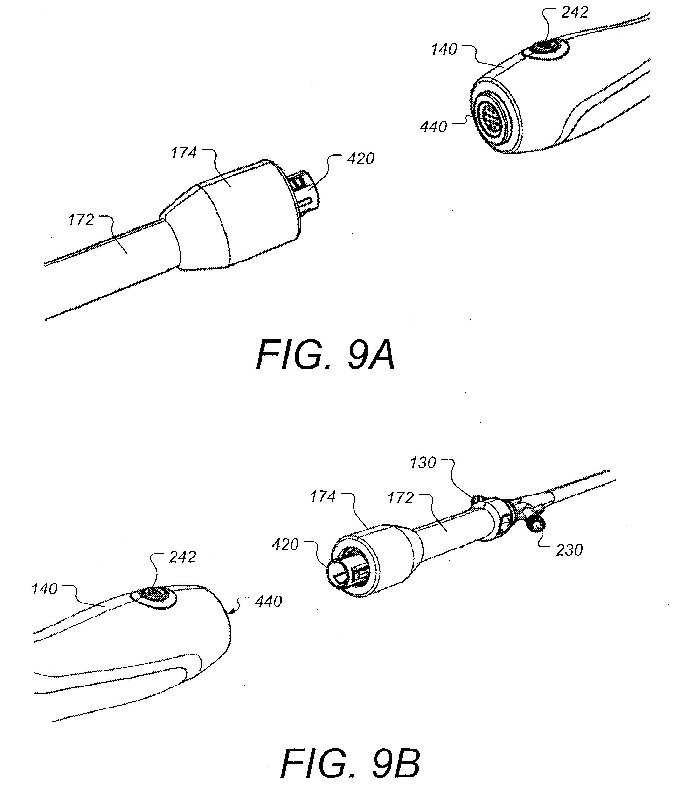

FIGs. 9A and 9B are perspective views showing further detail of connectors between single use and multiple use portions of an endoscopic system configured to deploy a surgical stent, according to some embodiments. The connector 420 has pins (not shown) that make electrical contact to sockets within connector 440 on the hand piece 140. According to some embodiments, the connectors 420 and 440 and mating collars surrounding them as illustrated are suitable for maintaining secure mechanical mating between the hand piece 140 of the multiple use portion (portion 104 shown in FIG. 1) and shaft 172 of the single use portion (portion 102 shown in FIG. 1).

FIG. 10 is a side view showing further detail of the distal tip of an endoscopic system configured to deploy a surgical stent, according to some embodiments. In the example shown the outer diameter of the inner cannula 420 (about 4.5 mm) is slightly larger than the outer diameter of the distal tip 110 (about 3.8 mm). There is a slight "step" or "lip" formed by the diameter change and the ring 710 formed at the distal end of stent 160 is configured with an inner diameter between the two sizes so that it fits around the distal tip 110 but cannot slide further proximally past the lip / step formed by the distal end of the inner cannula 420. According to some other embodiments other dimensions can be used and/or the step can be formed on the housing of the distal tip itself or upon the body of the inner cannula. According to some other embodiments, there is no diameter change or step and the stent 160 is held in place by friction forces alone.

FIGs. 11A-11C are diagrams illustrating further detail of a wire or rod used aid in deployment of a surgical stent, according to some embodiments. Wire 1100 is shown running through port 130 and through cannula 120 toward stent 160. According to some embodiments, the wire 1100 is positioned in the annular space between the inner cannula the outer sleeve of cannula 120. The distal end of wire 1100 has a fork or other suitable shape that allows for engagement with a portion on the proximal end of stent 160. The wire is suitably stiff to allow for the operator to exert a pushing force, in the distal direction. During deployment, shown in FIG. 11C, when the outer sleeve 422 is retracted proximally, the operator can manipulate the handle loop 1102 of wire 1100 to push or urge the stent off of the end of the inner cannula 420 as shown by the dashed arrows. According to some embodiments, wire 1100 can thus be used to ensure precision placement of the stent 160.

FIGs. 12A-12F are various views of an endoscopic system configured to remove a surgical stent, according to some embodiments. In this example, the distal region of the system 100 is shown configured for stent removal. The distal tip 110 includes three retrieval hooks 1210 that protrude distally and are shaped to engage with proximal loops 720 of stent 160. The outer sleeve 422 can be used to surround the hooks so that the hooks 1210 are partially or fully enclosed within the sleeve 422. As shown in FIG. 12A, outer sleeve 422, collar 170 and shaft 172 are configured to allow sleeve 422 to protrude distally past the distal tip 110 and hooks 1210. The system 100 includes single use portion 102 and multiple use portion 104. According to some embodiments, the multiple use portion 104 is identical to the portion 104 shown in FIGs. 1-4, but the single use portion 102 shown in FIGs. 12A-E is customized for retrieval / removal of stents. In this case there can be two separate cannula versions, one for deployment and one for retrieval of stents, each of which can be separately mated to a single multiple use portion that includes the hand piece and display.

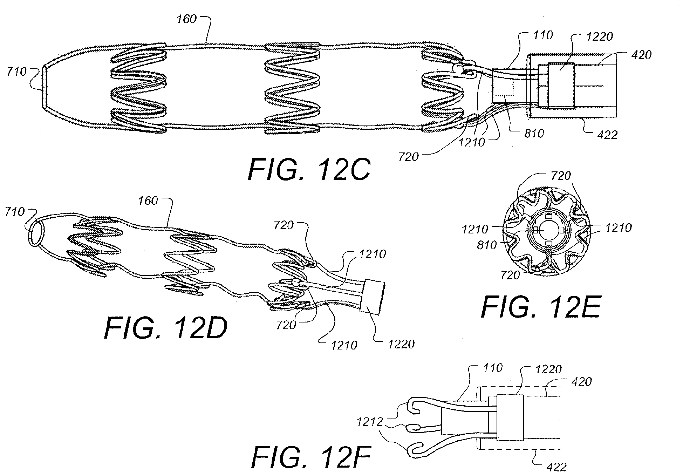

The position shown in FIG. 12A is when the sleeve 422 is in a proximally retracted position. FIG. 12B shows further detail of the hooks 1210 engaged with the proximal loops 720 of stent 160. Moving the sleeve 422 in a longitudinal direction relative to hooks 1210 allows hooks 1210 to expand and contract radially. Note that partially or fully enclosing the hooks 1210 is desirable during insertion of the cannula 422 through the urethra, for example (as shown in FIG. 13A). FIGs. 12C and 12D show further detail of the distal tip region. According to some embodiments, hooks 1210 can be made of stainless steel and fixed (e.g. welded) to a steel band 1220 that can in-tum be fixed to the distal end of inner cannula 420. According to some embodiments, the inner cannula 420 is also made of stainless steel and band 1220 and 1210 can be fixed to it by welding as well. FIG. 12E is a distal end view of the stent 160 being engaged by hooks 1210. Visible are the camera module 810 surrounded by LEDs on the distal tip of the retrieval device. According to some embodiments, other hook shapes can be used instead of a sphere on the end of a curved piece, as shown in FIGs. 12B-E. For example, in FIG. 12F, the hooks 1212 are shaped more as traditional hooks which may be suitable for ensuring a secure engagement with the proximal end of the stent during the removal process.

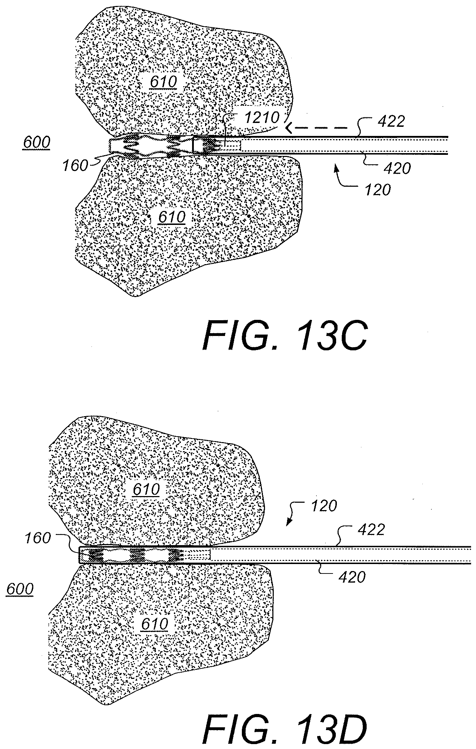

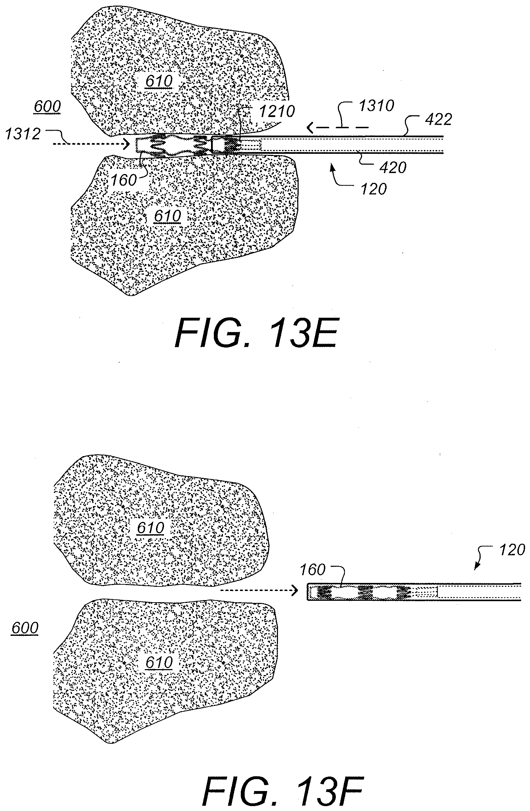

FIGs. 13A-13F are a series of diagrams illustrating methods for removing a prostatic stent from a male urethra, according to some examples not according to the claimed invention. In FIG. 13A, the cannula 120 is inserted through the urethra as indicated by the dashed arrow. The retrieval hooks 1210 are fully or nearly fully enclosed by the outer sleeve 422. In FIG. 13B, the tip of hooks 1210 are shown in position to engage with the proximal loops on stent 160 (loops 720 shown in FIGs. 7A and 7B). The precise positioning of the hooks and engagement of the loops on the stent is ensured by direct vision - using live video images captured by the camera module 810 in this distal tip and displayed to the operator on the display screen 150. In FIG. 13C, once the engagement of the hooks 1210 on the loops of the stent 160 is confirmed, the outer sleeve 422 is pushed distally as shown by the dashed arrow while the hooks 1210, distal tip and inner cannula 420 remain in a static position. The sleeve 422 forces the stent 160 into a compressed state. This can be accomplished by the operator pushing the collar distally while maintaining a steady position on the hand piece. In FIG, 13D, the sleeve 422 is shown fully extended distally such that the stent 160 is fully enclosed and compressed by sleeve 422. In FIG. 13F, the cannula 120, with the stent 160 securely held in the compressed state, is withdrawn from the urethra. According to some embodiments, once the engagement of the hooks 1210 on the loops of the stent 160 is confirmed, instead of maintaining a steady position on the hand piece as shown in FIGs. 13C and 13D, a combination of pulling (proximally) on the hand piece and pushing on the collar (distally) can be used. This is shown in FIG. 13E where the inner cannula 420 hooks 1210 and stent 160 are being pulled proximally as shown by dotted arrow 1312 while the sleeve 422 is being pushed distally as shown by dashed arrow 1310. According to yet another embodiment, once the engagement of the hooks 1210 on the loops of the stent 160 is confirmed the sleeve 422 can be maintained in a static location relative to the target tissues (prostate 610) while the inner cannula 420 and hooks 1210 are used to pull the stent 160 in a proximal direction until it is partially or fully enclosed within the stationary sleeve 422. This can be accomplished by the operator simultaneously pulling (proximally) on the hand piece 140 (shown in FIG. 12A) while maintaining position relative to the patient's body on the collar 170 (shown in FIG. 12A).

## Claims

1. An endoscope (100) for deploying and withdrawing a tubular stent (160) from a passage in a patient (620), comprising:
a handle (140) configured to be grasped by a user;
a shaft (172) extending distally relative to the handle;
an outer sleeve (422) extending distally from the shaft;
an inner cannula (420) configured to fit within the outer sleeve;
a collar (170) coupled with the outer sleeve and configured to move together therewith between a distal position in which distal ends of the inner cannula and the outer sleeve align and a proximal position by which a distal portion of the inner cannula protrudes distally from the outer sleeve;
a tubular stent (160);
said outer sleeve being configured to move relative to the handle and the inner cannula between a distal position and a proximal position;
wherein when the outer sleeve is in its proximal position a distal portion of the inner cannula protrudes distally from the outer sleeve and when the outer sleeve is in its distal position the tubular stent fits in a tubular space between distal portions of the outer sleeve and the inner cannula;
said tubular space is a tubular radial space and is configured to constrain the radially expandable tubular stent in a compressed state and said distal portion of the inner cannula is configured to engage the tubular stent to prevent proximal motion but allow distal motion of the tubular stent relative to the inner cannula, the tubular space matches the tubular stent in length such that when the outer sleeve is at its proximal position the tubular stent is not constrained by the outer sleeve and can expand radially to an expanded state and remain in said passage; and
a camera module (810) at the distal end of said inner cannula and a video screen attached to and mechanically supported by said handle and configured to show images of the passage and of the stent being deployed therein;
mechanical and electrical connectors (420, 440) between said shaft and said handle, wherein the shaft, collar, outer sleeve and cannula form a single-use stent-deployment portion of the endoscope and the handle and video screen form a reusable portion, and said connectors configured to release by hand, without tools, for disposal of said single-use portion after use thereof in a patient procedure;
further comprising a single-use stent removal portion that is configured to removably attach to said handle, in place of a stent deployment portion, and differs from a stent deployment portion by including plural hooks (1212) at a distal end of the inner cannula, which hooks are configured to fit between the inner cannula and the outer sleeve when the outer sleeve is in its distal position and to expand radially as the outer sleeve moves to its proximal position and to engage loops at a proximal portion of said tubular stent (160) to be removed.

2. The endoscope of claim 1, in which each of the single-use stent-deployment portion and the single-use stent-removal portion further comprises a housing (174) that has a proximal end connecting to said handle, a distal end connecting to said shaft, and an annular space (540) in which a proximal portion of the shaft moves between said distal and proximal position of the shaft.

3. The endoscope of claim 1, further including proximal and distal ports in said outer sleeve of the single-use stent-deployment portion and a fluid conduit through which fluid moves within and along a length of said outer sleeve between said ports.

4. The endoscope of claim 1, in which said inner cannula of the single-use stent-deployment portion has a distal end stepped down in diameter (110) to engage a matching diameter distal end of the stent to thereby prevent proximal motion but allow distal motion of the stent relative to the inner cannula.

5. The endoscope of claim 1, in which at least distal portions of the outer sleeve of the single-use stent-deployment portion and the inner cannula of the single-use stent-deployment portion are sufficiently flexible to bend when being inserted in curving portions of said passage.

## Patentansprüche

1. Endoskop (100) zum Setzen und Herausziehen eines röhrenförmigen Stents (160) aus einem Gang in einem Patienten (620), umfassend:
einen Handgriff (140), der konfiguriert ist, um von einem Benutzer ergriffen zu werden,
einen Schaft (172), der sich distal relativ zu dem Handgriff erstreckt;
eine äußere Hülse (422), die sich distal von dem Schaft erstreckt;
eine innere Kanüle (420), die konfiguriert ist, um in die äußere Hülse zu passen,
einen Bund (170), der mit der äußeren Hülse gekoppelt und konfiguriert ist, um sich zusammen mit dieser zwischen einer distalen Lage, in der distale Enden der inneren Kanüle und der äußeren Hülse fluchten, und einer proximalen Lage, in der ein distaler Teil der inneren Kanüle distal aus der äußeren Hülse herausragt, zu bewegen;
einen röhrenförmigen Stent (160);
wobei die genannte äußere Hülse konfiguriert ist, um sich relativ zu dem Handgriff und der inneren Kanüle zwischen einer distalen Lage und einer proximalen Lage zu bewegen;
bei dem, wenn sich die äußere Hülse in ihrer proximalen Lage befindet, ein distaler Teil der inneren Kanüle distal aus der äußeren Hülse herausragt und, wenn sich die äußere Hülse in ihrer distalen Lage befindet, der röhrenförmige Stent in einen röhrenförmigen Raum zwischen distalen Teilen der äußeren Hülse und der inneren Kanüle passt;
der genannte röhrenförmige Raum ein röhrenförmiger radialer Raum ist und zum Einschränken des radial ausdehnbaren röhrenförmigen Stents in einem komprimierten Zustand konfiguriert ist und der genannte distale Teil der inneren Kanüle konfiguriert ist, um mit dem röhrenförmigen Stent in Eingriff zu kommen, um eine proximale Bewegung zu verhindern, aber eine distale Bewegung des röhrenförmigen Stents relativ zu der inneren Kanüle zu ermöglichen, wobei der röhrenförmige Raum in seiner Länge mit dem röhrenförmigen Stent übereinstimmt, so dass, wenn sich die äußere Hülse in ihrer proximalen Lage befindet, der röhrenförmige Stent nicht von der äußeren Hülse eingeschränkt wird und sich radial in einen ausgedehnten Zustand ausdehnen und in dem genannten Gang verbleiben kann; und
ein Kameramodul (810) am distalen Ende der genannten inneren Kanüle und einen Videobildschirm, der an bzw. von dem genannten Handgriff angebracht ist und mechanisch gehalten wird und konfiguriert ist, um Bilder des Gangs und des darin gesetzten Stents zu zeigen;
mechanische und elektrische Verbinder (420, 440) zwischen dem genannten Schaft und dem genannten Handgriff, wobei der Schaft, der Bund, die äußere Hülse und die Kanüle einen Einweg-Stentsetzteil des Endoskops bilden und der Handgriff und der Videobildschirm einen wiederverwendbaren Teil bilden und die genannten Verbinder konfiguriert sind, um zum Entsorgen des genannten Einwegteils nach dessen Verwendung in einem Eingriff an einem Patienten ohne Werkzeuge von Hand gelöst zu werden,
ferner umfassend einen Einweg-Stententfernungsteil, der konfiguriert ist, um anstelle eines Stentsetzteils abnehmbar an dem genannten Handgriff angebracht zu werden, und der sich von einem Stentsetzteil dadurch unterscheidet, dass er mehrere Haken (1212) an einem distalen Ende der inneren Kanüle beinhaltet, wobei die Haken konfiguriert sind, um zwischen die innere Kanüle und die äußere Hülse zu passen, wenn sich die äußere Hülse in ihrer distalen Lage befindet, und sich beim Bewegen der äußeren Hülse in ihre proximale Lage radial auszudehnen und in Schlaufen an einem proximalen Teil des genannten zu entfernenden röhrenförmigen Stents (160) in Eingriff zu kommen.

2. Endoskop nach Anspruch 1, bei dem jeder des Einweg-Stentsetzteils und des Einweg-Stententfernungsteils ferner ein Gehäuse (174) umfasst, das ein mit dem genannten Handgriff verbundenes proximales Ende, ein mit dem genannten Schaft verbundenes distales Ende und einen ringförmigen Raum (540), in dem sich ein proximaler Teil des Schafts zwischen der genannten distalen und proximalen Lage des Schafts bewegt, aufweist.

3. Endoskop nach Anspruch 1, das ferner eine proximale und eine distale Öffnung in der genannten äußeren Hülse des Einweg-Stentsetzteils und eine Flüssigkeitsleitung beinhaltet, durch die sich Flüssigkeit innerhalb und entlang einer Länge der genannten äußeren Hülse zwischen den genannten Öffnungen bewegt.

4. Endoskop nach Anspruch 1, bei dem die genannte innere Kanüle des Einweg-Stentsetzteils ein distales Ende aufweist, das im Durchmesser (110) abgestuft ist, um mit einem distalen Ende des Stents mit passendem Durchmesser in Eingriff zu kommen, um dadurch eine proximale Bewegung zu verhindern, aber eine distale Bewegung des Stents relativ zur inneren Kanüle zu ermöglichen.

5. Endoskop nach Anspruch 1, bei dem zumindest distale Teile der äußeren Hülse des Einweg-Stentsetzteils und der inneren Kanüle des Einweg-Stentsetzteils ausreichend flexibel sind, um sich zu biegen, wenn sie in gekrümmte Teile des genannten Gangs eingeführt werden.

## Revendications

1. Endoscope (100) destiné à déployer et à retirer une endoprothèse tubulaire (160) d'un passage chez un patient (620), comprenant :
une poignée (140) configurée pour être saisie par un utilisateur ;
une tige (172) s'étendant de manière distale par rapport à la poignée ;
un manchon externe (422) s'étendant de manière distale depuis la tige ;
une canule interne (420) configurée pour s'ajuster au sein du manchon externe ;
un collier (170) couplé avec le manchon externe et configuré pour se déplacer ensemble avec celui-ci entre une position distale dans laquelle les extrémités distales de la canule interne et du manchon externe s'alignent et une position proximale par laquelle une partie distale de la canule interne dépasse du manchon externe ;
une endoprothèse tubulaire (160) ;
ledit manchon externe étant configuré pour se déplacer par rapport à la poignée et la canule interne entre une position distale et une position proximale ;
dans lequel quand le manchon externe est dans sa position proximale une partie distale de la canule interne dépasse de manière distale du manchon externe et quand le manchon externe est dans sa position distale l'endoprothèse tubulaire s'ajuste dans un espace tubulaire entre les parties distales du manchon externe et de la canule interne ;
ledit espace tubulaire est un espace radial tubulaire et est configuré pour contraindre l'endoprothèse tubulaire capable de déploiement de manière radiale dans un état comprimé et ladite partie distale de la canule interne est configurée pour entrer en prise avec l'endoprothèse tubulaire pour empêcher un mouvement proximal mais pour permettre un mouvement distal de l'endoprothèse tubulaire par rapport à la canule interne, l'espace tubulaire correspond en longueur à l'endoprothèse tubulaire de sorte que quand le manchon externe est dans sa position proximale l'endoprothèse tubulaire n'est pas contrainte par le manchon externe et peut se déployer de manière radiale vers un état déployé et rester dans ledit passage ; et
un module de caméra (810) au niveau de l'extrémité distale de ladite canule interne et un écran vidéo attaché à et supporté mécaniquement par ladite poignée et configuré pour montrer des images du passage et de l'endoprothèse en cours de déploiement dans celui-ci ;
des connecteurs mécaniques et électriques (420, 440) entre ladite tige et ladite poignée, dans lequel la tige, le collier, le manchon externe et la canule forment une partie de déploiement d'endoprothèse à usage unique de l'endoscope et la poignée et l'écran vidéo forment une partie réutilisable, et lesdits connecteurs configurés pour être libérés à la main, sans outils, pour éliminer ladite partie à usage unique après l'utilisation de celle-ci dans une procédure de patient ;
comprenant en outre une partie de détachement d'endoprothèse à usage unique qui est configurée pour s'attacher de manière détachable à ladite poignée, à la place d'une partie de déploiement d'endoprothèse, et diffère d'une partie de déploiement d'endoprothèse par l'inclusion de plusieurs crochets (1212) au niveau d'une extrémité distale de la canule interne, lesquels crochets sont configurés pour s'ajuster entre la canule interne et le manchon externe quand le manchon externe est dans sa position distale et pour se déployer de manière radiale alors que le manchon externe se déplace vers sa position proximale et pour entrer en prise avec des boucles au niveau d'une partie proximale de ladite endoprothèse tubulaire (160) devant être détachée.

2. Endoscope selon la revendication 1, dans lequel chacune de la partie de déploiement d'endoprothèse à usage unique et de la partie de détachement d'endoprothèse à usage unique comprend en outre un logement (174) qui a une extrémité proximale se connectant à ladite poignée, une extrémité distale se connectant à ladite tige, et un espace annulaire (540) dans lequel une partie proximale de la tige se déplace entre ladite position distale et ladite position proximale de la tige.

3. Endoscope selon la revendication 1, incluant en outre des orifices proximal et distal dans ledit manchon externe de la partie de déploiement d'endoprothèse à usage unique et un conduit de fluide à travers lequel du fluide se déplace au sein et le long d'une longueur dudit manchon externe entre lesdits orifices.

4. Endoscope selon la revendication 1, dans lequel ladite canule interne de la partie de déploiement d'endoprothèse à usage unique a une extrémité distale dont le diamètre (110) se réduit pour entrer en prise avec une extrémité distale à diamètre correspondant de l'endoprothèse pour empêcher ainsi un mouvement proximal mais permettre un mouvement distal de l'endoprothèse par rapport à la canule interne.

5. Endoscope selon la revendication 1, dans lequel au moins des parties distales du manchon externe de la partie de déploiement d'endoprothèse à usage unique et de la canule interne de la partie de déploiement d'endoprothèse à usage unique sont suffisamment souples pour plier quand elles sont insérées dans des parties courbées dudit passage.
